# EUROPEAN PATENT SPECIFICATION

(11) **EP 0 755 436 B1**
(45) Date of publication and mention of the grant of the patent: **07.11.2001**
(21) Application number: 95914370.2
(22) Date of filing: 30.03.1995
(51) Int. Cl.: C12M 1/34, G01N 33/18

(54) **BIOFILM DEVICE FOR THE MONITORING OF MICROBIAL DISTURBANCES IN INDUSTRIAL PROCESS WATERS**
VORRICHTUNG MIT EINEM BIOFILM ZUR BEOBACHTUNG VON MIKROBIELLEN STÖRUNGEN IN INDUSTRIELLEM PROZESSWASSER
DISPOSITIF D'EXAMEN DE FILM BIOLOGIQUE DESTINE A LA SURVEILLANCE DE PERTURBATIONS MICROBIENNES DANS DES EAUX DE TRAITEMENT INDUSTRIEL

(30) Priority: 31.03.1994 FI 941539
(43) Date of publication of application: 29.01.1997
(73) Proprietor: KEMIRA CHEMICALS OY, 00101 Helsinki (FI)
(72) Inventor: VÄÄTÄNEN, Pentti, 65370 Vaasa (FI)
(74) Representative: Risku, Ira Marjatta
(86) International application number: FI9500174
(87) International publication number: WO9527039

(56) References cited:
- WO-A-93/01497
- US-A- 4 945 758
- US-A- 5 049 492

## Description

The invention relates to a modern so-called biofilm device for the microbiological monitoring of industrial process waters. The device of this invention is particularly advantageous when microbial disturbances of paper industry are monitored. By means of the biofilm device it is namely possible to predict runnability problems caused by microbes. It can also be used for the selection of control methods in the paper mill itself.

Biofilm is a term generally used in the field for a deposit formed by microbes on surfaces.

The worst microbial problems in the paper and board manufacture are the runnability problems caused by microbial deposits i.e. the formations of holes and patches in the product, web breaks as well as drainage disturbances caused by blockages of machine felts. For the monitoring of these problems determination of the bacterial contents of circulation water samples is generally used.

The freely floating common bacteria of the circulation waters cannot, however, give reliable indication of the runnability problems since especially the microbial deposits cause runnability problems - not separate bacteria in the circulation water - and, on the other hand, the deposits are formed on the surfaces, not in the circulation waters. Accordingly investigations of bacterial contents of the circulation waters are useless with respect to the prediction and monitoring of the runnability problems of paper and board machines.

Essentially more useful than the foregoing is to determine in circulation waters certain microbes causing deposits, such as filamentous bacteria, filamentous yeasts and molds and slime-forming bacteria. In this way one can indirectly monitor biofilm-forming microbes that possibly have come loose from surfaces to the circulation water. If there are plenty of such bacteria in the circulation waters, it might already be too late to control microbial problems and the runnability of the paper machine may already have weakened.

Thus, the best way to avoid runnability problems is to monitor problem microbes directly from the biofilms formed on the surfaces. During the normal production of paper and board machines this is, however, difficult because the deposit samples can be handily taken only in the beginning of the shutdowns just before the washing of the machinery.

The aim of the present invention is to remove the above-mentioned drawbacks and to develop a device with which it is possible to predict runnability problems caused by microbes particularly in paper and board mills. By the device of the invention the microbiological status of the surfaces of pipings and containers can be found out also during the normal production without stopping the run of the machines.

The characteristic features of the invention are presented in the attached patent claims.

The biofilm device of the invention consists of a cylindrical body and steel plates placed symmetrically in regard to each other and with respect to the flow of the circulation water inside the body, on the surfaces of which the microbes as well as other material, such as fibrils, fibers, pitch and pigment particles can attach from the side streams of the circulation water or a container. The symmetrical arrangement of the steel plates is an absolute prerequisite to make the steel plates comparable taken at various times into investigation.

There has been biofilm devices in use previously. Pedersen, Appl. and Environ. Microbiol., vol. 43(1)(1982), p. 6-13, has developed a device for water research, in which small and easily breakable cover glasses are placed in a box above each other as "shelves". Hereby, the cover glasses are not in a symmetrical position with respect to the flow, and, thus, they are not equal with each other. Another disadvantage of the device is the fragility of the cover glasses. This type of a device cannot be applied for the biofilm examinations of process industry.

The Robbins device (Ruseska I. et al., Oil & Gas Journal, vol. 80 (1982), p. 253-264) does not fulfil the symmetry requirement either because removable sample plates are placed after each other along a pipe. The last plate is, in the theory, in the most disadvantageous position compared with the first plate as regards the flow. The size of the sample plates of the Robbins device is all too small to carry out microbiological analyses of the biofilms deposited on the plates.

Conkey J., TAPPI Proceedings (1984), p. 115-120, has developed a tubular device, the principle of operation of which is fully different from that of the biofilm device of the present invention. The intention of the device is, namely, to indirectly monitor the amount of the material attaching on the surfaces of the pipe by measuring the flow resistance. The Conkey device does not contain detachable sample plates for laboratory investigations.

The new device of the present invention is preferably prepared from acid-proof steel, which is a generally used pipe and container material in paper industry. This is important in a biofilm device because microbes attach differently for example to glass than to steel. To secure the reliability of the device the same material should be used in it as in the equipment to be investigated. The device of the invention and its steel plates tolerate fairly great pressures and strong flows contrary to, for example, the thin cover glass plates of the above-mentioned Pedersen device.

The advantages of the biofilm device of the invention as compared with other devices are in summary the symmetric position of the sample plates made of steel in regard to each other and the flow-through, an adequate size of the sample plates for various microbiological analyses, the strength of the structure and in addition to this the suitability of the steel plates as such for epifluorescence microscopy.

The biofilm device of the invention is also suitable to be used for testing the efficiency of biocides in the process of the mill. So far the testing and choice of biocides has taken place in laboratories, where it is difficult to simulate the dynamic conditions of circulation water. The device of the invention is also suitable both for testing biocides and for testing dispersing agents and washing chemicals.

The device of the invention can be connected with any side stream of the circulation water or container in a paper or board mill or, for example, with a flow taken from the wet end. The flow-through of the device is preferably adjusted to the range of 0.5 to 100 liters/min. The sample plates are preferably kept in the flow to be investigated for about 1 to 90 days.

In the following the invention is described in more detail by referring to a figure describing a biofilm device of the invention in a partial side section and a cut-through of the biofilm device at point A-A of the steel plates perpendicularly against the vertical axis.

The device consists of a cylindrical body 1 made of acid-proof steel, the ends of which have been formed conical. Sample plates 2 made of acid-proof steel have been placed inside the body so that they lie symmetrically around the middle axis of the body 1 parallel to the axis. The body 1 and the steel plates 2 can also be made of some other durable material. The body 1 of the device is hinged 3 in the middle or otherwise openable to install the sample plates and to remove them. Inside the body 1 there is, for example, a hoop-formed holder 4, to which the lower end of the sample plates 2 can be placed for the symmetrical installing of the sample plates. The plates are fixed at their upper end of the sample plates 2 by a locking means (not shown) which can be, for example, a hoop-formed holder.

As can be seen from the cross-section of the cylindrical body 1 the steel plates have been installed radially around the middle axis of the body parallel with the axis, whereby all plates are equal with each other. The number of the sample plates can be any number, provided the flow resistance will not grow harmfully high.

The device comprises a valve (not shown) connected with the inlet for the opening, closing and regulation of the flow-through.

In the following the operation of the device of the invention is described by means of examples. Experiments have been made both in laboratory and in industrial scale.

### Example 1

In laboratory, ground wood diluted with a very weak nutrient solution (MYGP) in the ratio 1:5 was circulated through two biofilm devices according to the invention.

The composition of the MYGP solution was the following:

| | |
|---|---|
| Bacto malt extract | 3g |
| Bacto yeast extract | 3g |
| Bacto peptone | 5g |
| D glucose | 10g |
| distilled water | 1000ml |

dilution in the ratio 1:40 with water.

A yeast isolated from the broke of a paper mill was chosen as the microbe to be investigated. It was added into the mixture to be circulated to the concentration 10,000 cfu (colony-forming units) per ml. To the circulation of the first device 20 ppm of biocide Fennosan M 9 was added and the second biofilm device served as a control. The steel plates of the devices were taken for investigation after three and six days and the number of yeast cells per sq. cm was determined. The results are presented in Table 1.

**Table 1**

| Time days | Yeasts attached to steel plates (cfu/cm²) | |
|---|---|---|
| | Control | Fennosan M 9 |
| 3 | 140 000 | 37 000 |
| 6 | 1 600 000 | 11 000 |

The results show that after 6 days lots of yeasts had attached to the steel plates of the control device and had grown there well so the device functioned according to expectations. In the presence of a biocide the attachment of yeast cells remained fractional i.e. at about 0.3%.

### Example 2

The laboratory experiment was carried out in the same way as in example 1. Yeast cells were added to a concentration of 290,000 cfu/ml. The yeast was from a board mill. Hydrogen peroxide (50ppm), which is known for its ability to prevent formation of a biofilm, was used as an inhibitive chemical. The results are presented in Table 2.

**Table 2**

| Time days | Yeasts attached to the steel plates (cfu/cm²) | |
|---|---|---|
| | Control | Hydrogen peroxide |
| 2 | 170 000 | 110 000 |
| 5 | 2 000 000 | 80 000 |
| 12 | 2 000 000 | 40 000 |

Also in this case the biofilm device functioned well. Lots of yeast cells (2 million cfu/cm²) had attached to the steel plates of the control device in 5 and 12 days, whereas the number of yeast cells in the presence of hydrogen peroxide remained, when lowest, at 2% of the control.

### Example 3

The idea of this example is to show the significance of the biofilm forming microbes harming the runnability of a paper machine. The head box and the wire tank of a paper machine were chosen as the sampling points. A biofilm device according to the invention was connected with the wire tank. Harmful molds were determined both from the biofilms of the surfaces and from the circulation water. The results are given in table 3.

**Table 3**

| Sampling point | Number of molds (cfu/ml) | |
|---|---|---|
| | Circulation water | Biofilm |
| Head box | <100 | 1400 |
| Wire tank | <100 | 5400 |

The results show that molds harmful to the runnability could be found only in the biofilms of the surfaces, but not at all in the adjacent circulation water. Lots of molds could be found on the steel plates of the biofilm device connected with the wire tank. This experiment shows the utility of the biofilm device according to the invention in a mill.

### Example 4

In this test the biofilm device was connected with the white water tank of a paper mill. The aim was to test the function of the device and the possible attachment of harmful microbes on its steel plates. The steel plates were installed in the device on different days. The results are given in Table 4.

**Table 4**

| Time days | Bacteria (cfu/cm²) | Fungi (cfu/cm²) |
|---|---|---|
| 12 | 3 000 | <30 |
| 21 | 24 000 | 270 |
| 22 | 140 000 | 5 400 |
| 34 | 730 000 | 32 000 |

The results show that bacteria and fungi attached in the greater extent the longer the steel plates had been in the side stream of the white water tank. At a microscope the fungi were found to be filamentous i.e. potential deposit formers. Also some of the bacteria were filamentous.

In summary, on the basis of the test results, it can be said that the function of the biofilm device according to the invention is reliable. By means of the device new information from the community structure and amount of microbes attaching to the surfaces may be obtained under production conditions. This information can be utilized in combatting microbes harmful to the runnability and in the optimization and scheduling of washings of the production machines.

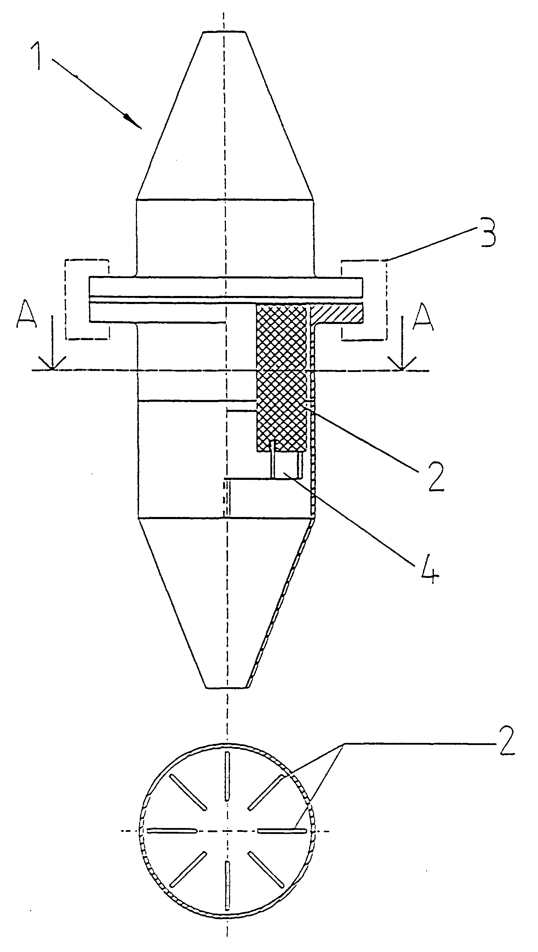

## Claims

1. Device for the monitoring of biofilm-forming microbes in industrial process waters, **characterized in that** the device consists of a cylindrical, openable body (1) made of acid-proof steel or other durable material, sample plates (2) made of acid-proof steel or other durable material, placed symmetrically in respect to each other and to the flow of the water, inside the body (1) parallel to its middle axis, and holders (4) placed inside the body (1) for the mounting and holding of the sample plates (2).

2. Device according to claim 1, wherein the device further comprises a valve for the opening, closing and regulation of the flow-through.

3. Device according to claim 1 or 2, wherein it is connected with the circulation water of a mill or the side flow of a tank or, for example, with a flow taken from the wet end of a paper or board-mill.

4. Device according to any of claims 1 to 3, wherein the flow-through of the device is adjusted to the range of 0.5 to 100 liters/min.

5. Device according to any of claims 1 to 4, wherein the sample plates are kept in the flow to be investigated for 1 to 90 days.

## Patentansprüche

1. Vorrichtung zur Überwachung von Biofilm-bildenden Mikroben in industriellen Prozesswässern, **dadurch gekennzeichnet, dass** die Vorrichtung aus einem zylindrischen, zu öffnenden Körper (1), der aus säurebeständigem Stahl oder einem anderen beständigen Material hergestellt ist; Probenplatten (2), die aus säurebeständigem Stahl oder einem anderen beständigen Material hergestellt sind, die in Bezug auf einander und auf den Fluss des Wassers symmetrisch im Innern des Körpers, parallel zu dessen Mittelachse, angeordnet sind; und Halter (4), die für das Montieren und Halten der Probenplatten (2) im Innern des Körpers (1) angeordnet sind; besteht.

2. Vorrichtung nach Anspruch 1, wobei die Vorrichtung weiter ein Ventil für das Öffnen, Schliessen und Regulieren des Durchstromes umfasst.

3. Vorrichtung nach Anspruch 1 oder 2, wobei sie mit dem Zirkulationswasser einer Mühle oder dem Nebenstrom eines Tankes oder beispielsweise mit einem Fluss, der von der Nasspartie einer Papier- oder Kartonmühle entnommen wurde, verbunden ist.

4. Vorrichtung nach einem der Ansprüche 1 bis 3, wobei der Durchfluss der Vorrichtung auf den Bereich von 0,5 bis 100 Liter/Min. eingestellt ist.

5. Vorrichtung nach einem der Ansprüche 1 bis 4, wobei die Probenplatten für 1 bis 90 Tage in dem zu untersuchenden Fluss gehalten werden.

## Revendications

1. Dispositif pour la surveillance de microbes formateurs de films biologiques dans des eaux de traitement industriel, **caractérisé en ce que** le dispositif est constitué d'un corps cylindrique ouvrable (1) constitué d'un acier résistant aux acides ou d'un autre matériau durable, des plaques échantillon (2) constituées d'un acier résistant aux acides ou d'un autre matériau durable, placées symétriquement les unes par rapport aux autres et par rapport à l'écoulement de l'eau, à l'intérieur du corps (1) parallèle à son axe médian, et des dispositifs de maintien (4) placés à l'intérieur du corps (1) pour le montage et le maintien des plaques échantillon (2).

2. Dispositif selon la revendication 1, dans lequel le dispositif comporte en outre un clapet pour l'ouverture, la fermeture et la régulation du flux traversant.

3. Dispositif selon la revendication 1 ou 2, dans lequel il est relié à l'eau de circulation d'un broyeur ou à l'écoulement latéral d'une cuve ou, par exemple, à un écoulement pris à partir de l'extrémité humide d'un broyeur de papier ou de carton.

4. Dispositif selon l'une quelconque des revendications 1 à 3, dans lequel l'écoulement traversant du dispositif est ajusté dans l'intervalle de 0.5 à 100 litres/mn.

5. Dispositif selon l'une quelconque des revendications 1 à 4, dans lequel les plaques échantillon sont maintenues dans l'écoulement pour faire l'objet d'une étude pendant 1 à 90 jours.
